# EUROPEAN PATENT SPECIFICATION

(11) **EP 0 446 843 B1**
(45) Date of publication and mention of the grant of the patent: **04.06.1997**
(21) Application number: 91103702.6
(22) Date of filing: 11.03.1991
(51) Int. Cl.: G01N 11/00

(54) **Apparatus and method for evaluating phase change of emulsion**
Verfahren und Vorrichtung zur Bestimmung der Phasenveränderung einer Emulsion
Procédé et dispositif d'évaluation du changement de phare d'une émulsion

(30) Priority: 13.03.1990 JP 61883/90; 08.06.1990 JP 151132/90; 28.06.1990 JP 170790/90; 22.01.1991 JP 5949/91
(43) Date of publication of application: 18.09.1991
(73) Proprietor: RIKAGAKU KENKYUSHO, Wako-shi Saitama-ken (JP)
(72) Inventor: Date, Munehiro, c/o Rikagaku Kenkyusho, Saitama-ken (JP); Inaba, Yukio, Pieru Mejirokan, Tokyo (JP)
(74) Representative: Herrmann-Trentepohl, Werner, Dipl.-Ing.

(56) References cited:
- DE-A- 2 706 855
- GB-A- 2 013 903
- US-A- 4 095 461
- US-A- 4 512 182
- US-A- 4 862 735
- IBM TECHNICAL DISCLOSURE BULLETIN. vol. 26, no. 12, May 1984, NEW YORK US pages 6471 - 6472; R.L. IMKEN: "Analysis of cure behaviour"

## Description

### Field of the Invention

The present invention relates to an apparatus and a method for evaluating phase change of an emulsion.

### Background of the Invention

Emulsions are mixtures of two or more incompletely miscible liquids such as oil and water. There are many kinds of emulsions in use in the modern world. Some, such as milk, mayonnaise, creams for foods, cosmetic creams and the like, are used in the home. Others, such as paints, adhesives, drugs, pesticides, asphalt and the like, are utilized in industry.

If an emulsion is left to stand without agitation for a long period, it will generally separate into its two or more constituent liquids by creaming, cohesion and coalescence of its dispersed droplets. It is therefore important for industrial use of emulsions to be able to evaluate their chemical stability as well as their mechanical properties such as hardness, fluidity and deformability.

Some emulsions such as cosmetic creams, nutrient creams and butters are not stable during the use, and the evaluation of such emulsions has conventionally depended on tactile tests conducted by skilled panelers. Although human sensitivity is very high and such evaluations are generally accurate, they involve some disadvantages. Specifically, they are strongly influenced by the panelers' physical condition individual preferences, the test environment, and so on. Therefore, the data sometimes vary widely and are not always reliable.

From DE-A-27 06 855, there is known a rheometer measuring the absolute viscosity of an emulsion being present in one particular phase.

US-A-4,095,461 shows a rheometer by which also the viscous elastic properties of polymeric materials or other materials like foodstuffs, petrochemicals is measured.

The apparatuses known from DE-A-27 06 855 and US-A-4,095,461 are rheometers including an upper and lower part, where the upper part is fixed during the measurement.

GB-A-2 013 903 refers to the testing of rheological properties of a fluid between a pair of members having cooperating faces.

The above state of art being well known in the context of rheometers.

### Summary of the Invention

It is therefore an object of the invention to provide an apparatus and a method for evaluating phase change of emulsions, which can continuously analyze changes in the mechanical properties of the emulsion during phase change and can provide objective evaluations not dependent on the physical sensations of humans.

The present invention provides an apparatus for evaluating phase change of an emulsion according to claim 1.

The present invention also provides a method for evaluating phase change of an emulsion according to claim 10.

The above and other objects and features of the present invention will become apparent from the following description of preferred embodiments taking reference to the accompanying drawings.

### Brief Description of the Drawings

Figure 1 is a schematic view of an apparatus in accordance with one embodiment of the present invention.

Figure 2 is a schematic view of an apparatus in accordance with another embodiment of the present invention.

Figure 3 is a schematic view of an apparatus in accordance with another embodiment of the present invention.

Figure 4 illustrates the relationship between the measured shearing force and the microscopic state of a commercial cosmetic cream "M".

Figure 5 illustrates the relationship between the number of reciprocations and the measured shearing force in the case of commercial nutrient creams "A"and "B".

Figure 6 illustrates the relationship between the number of reciprocations and the measured shearing force in the case of commercial cleansing creams "C" and "D".

Figure 7 illustrates the relationship between the number of reciprocations and the measured shearing force in the case of commercial nutrient cream "A", in which four different films are used.

Figure 8 illustrates the relationship between the number of rotations and the measured shearing force in the case of a commercial nutrient cream "G".

Figure 9 illustrates the relationship between the number of rotations and the measured shearing force in the case of a commercial natural butter "YB".

### Description of the Preferred Embodiments

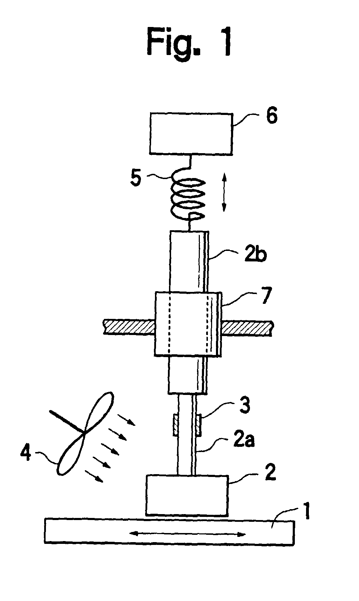

Figure 1 shows an apparatus in accordance with a first embodiment of the present invention.

The apparatus comprises a sliding part or movable plate 1 horizontally supported by bearings (not shown) and reciprocated parallel to a surface thereof (referred to herein as the "sliding surface") by a well-known drive mechanism (not shown). A film of polymer substance is preferably attached to the sliding surface. The emulsion to be evaluated is applied to the sliding surface, or to the attached film. The apparatus preferably includes a temperature controlling means (not shown) to control the temperature of the movable plate 1 between -20°C and 100°C.

The apparatus further comprises a pushing part 2 rigidly connected to a vertical shaft 2a which is in turn connected to an enlarged shaft 2b extending vertically upward. A film of polymer substance is preferably attached to the bottom surface of the pushing part 2. The shaft 2b is vertically slidably supported by a bearing portion 7 fixed to a stationary part(not shown). The bottom end of a vertically oriented coil spring 5 is attached to the top end of the shaft 2b and the top end of the spring 5 is attached to the bottom end of an up-and-down mechanism 6. The body of the up-and-down mechanism 6 is disposed on a stationary part(not shown) and the mechanism is adapted to move the shafts 2a and 2b up and down through the spring 5. The force of the spring 5 pushes the pushing part 2 or the attached film against the sliding surface of the movable plate 1 or the attached film to which the emulsion has been applied.

The apparatus further comprises a sensor means or strain gauge 3 attached to the surface of the shaft 2a and adapted to measure the force imparted to the pushing part 2 in the sliding direction. The sensor means need not necessarily be a strain gauge and any of various other well-known measuring devices can alternatively be used for measuring the force. A fan 4 is preferably positioned laterally of the pushing part 2 for blowing air onto the emulsion to volatilize its volatile components.

The procedure for using the apparatus is as follows.
(1) the emulsion is applied to the sliding surface of the movable plate 1 or to the attached film thereof,
(2) the movable plate 1 is reciprocated in parallel with the sliding surface by the drive mechanism (not shown),
(3) the pushing part 2 and the attached film thereof are pushed against the sliding surface by the up-and-down mechanism 6 with the emulsion present between the pushing part 2 and the sliding surface or between the attached films thereof,
(4) the force imparted to the pushing part 2 in the sliding direction is measured by the sensor 3, and
(5) phase change of the emulsion is evaluated from changes in the measured value.

The evaluation can be made by comparing the measured force with standard data, as shown later in the examples.

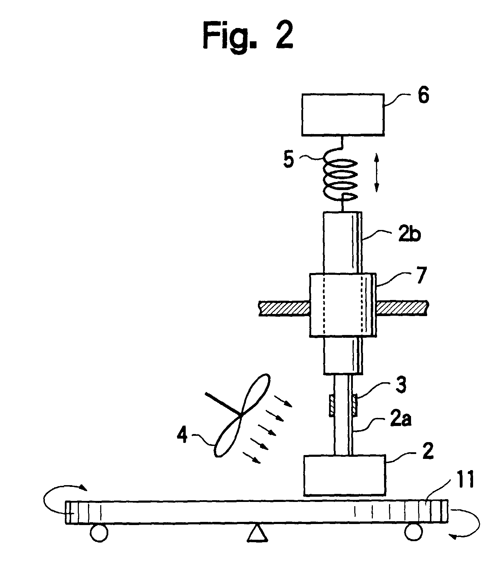

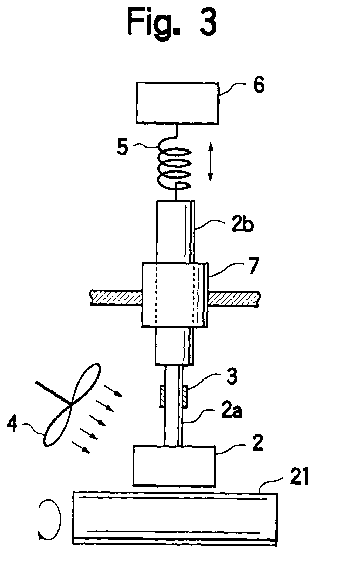

Figures 2 and 3 show two other apparatuses in accordance with the present invention.

In the apparatus of Figure 2, the sliding part is a disc 11 supported horizontally by bearings (not shown) and rotated around the axis thereof by a well-known drive mechanism (not shown).

In the apparatus of Figure 3, the sliding part is a cylidrical drum 21 supported by bearings (not shown) and rotated around the axis thereof by a well-known drive mechanism (not shown). The other parts of the apparatuses of Figures 2 and 3 are same as those of the first embodiment.

The sliding part and the pushing part preferably have high chemical stability, thermal shock resistance, mechanical shock resistance, and abrasion resistance. They are preferably made of a metal, ceramic, polymer substance, or a composite thereof.

When a metal is used it can be used either without any treatment or as coated with another metal by vapor depositions or plating.

Polymer substances can include addition polymers of vinyl compounds such as polyethylene, polystyrene, poly(meth)acrylic acid, poly(meth)acrylic ester, polyvinyl acetate, polyacrylonitrile and polyhalogenated vinyl, interpolymers of fluorinated vinyl compound with ethylene, vinylidene cyanide and the like, chemical compounds including fluorine such as polytrifluoroethylene, polytetrafluoroethylene and polyhexafluoropropylene, polyesters, polycarbonates and polyethers such as polyamide, polyimide, polyurethane and polyethylene terephthalate, and heat-hardening resins such as unsaturated polyester, epoxy and bakelite. The polymer substaces can be used either without any treatment for the surfaces of the sliding part and the pushing part, or after being subjected to one of the processes described below in response to their intended use.

When films of polymer substances are attached to the opposed surfaces of the sliding part and the pushing part, these films can be used either without any treatment or after being subjected to alkaline metal solvent treatment, treatment by the alkaline metal amalgam process, chemical etching by, for example, the electrolylic process, vapor deposition of silver, aluminium or the like, vacuum discharge treatment, sputter etching (coating), corona discharge treatment, graft treatment, plasma spray coating, sandblast treatment, chemical treatments for making films porous, and the like.

The substance to be used is preferably selected by comparing the results of organoleptie test with the test results obtained by the apparatus according to the invention. Generally chemically etched or sputter-etched films including fluorine are most preferable.

### Example 1

Sputter-etched films were obtained by sputtering PTFE virgin films by use of a sputtering apparatus. The original thickness of the films was about 15 µ m. SEM (scanning electron microscope) observation of the films showed it to have many fibres of 0.5 to 1.0 µ m length uniformly distributed over the surfaces. The surfaces were hydropihlic. The films were attached to the opposed surfaces of the movable plate 1 and the pushing part 2 shown in Figure 1.

A commercial cosmetic cream "M" was applied to the sliding surface of the movable plate 1 and tested by the apparatus according to the procedure described earlier. The sliding rate of the movable plate 1 was 9 reciprocations per minute and the number of reciprocations was 230.

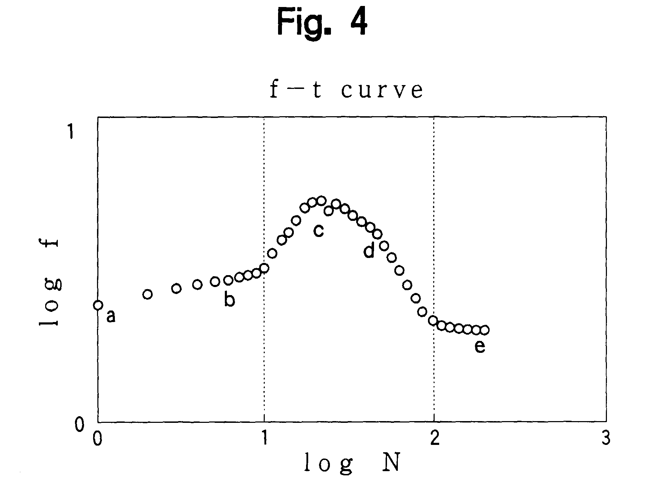

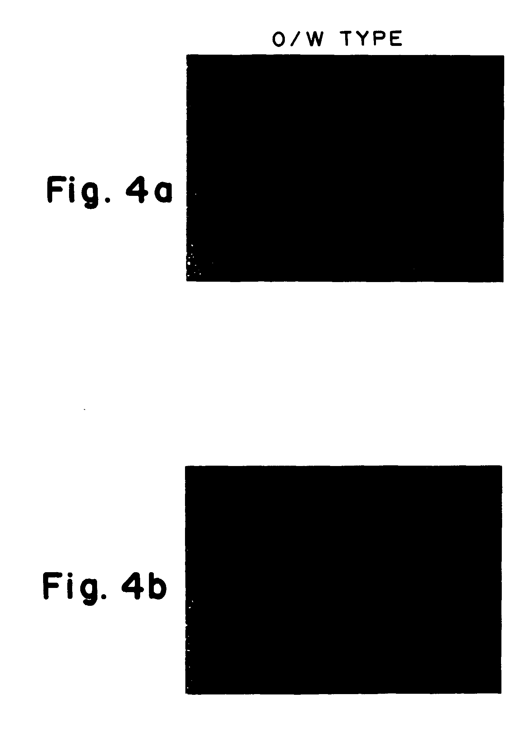

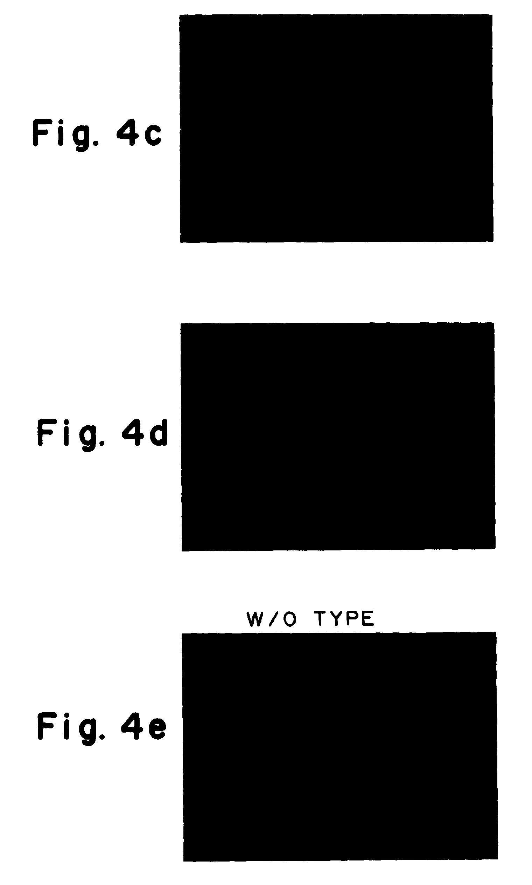

During the test, the force imparted to the pushing part 2 in the sliding direction was measured by the strain gauge 3. In addition, four samples of the emulsion were taken during the test and observed by an optical microscope. The results are shown in Figure 4 and Figure 4a through 4e.

In Figure 4, the horizontal axis represents the logarithm (log N) of the number of reciprocations of the movable plate 1 and the vertical axis represents the logarithm (log f) of the measured shearing force. Points "a","b","c","d" and "e" in the same figure are the points at which samples were taken during the test. The five microphotographs "a" through "e" in Figure 4a through 4e correspond to the sampling points.

As is apparent from these microphotographs, the cream was an oil-in-water type (hereinafter called O/W type) emulsion at point "a" (the starting point). At point "b" it was still of O/W type but had partially changed to water-in-oil-in-water type (W/O/W type). Between points "c" and "d", the phase was in the process of changing from O/W type to water-in-oil type (W/O type). At point "e", the phase had fully changed to W/O type. Accordingly, the cream was found to be an emulsion which could be changed from O/W type to W/O type.

A comparison of these microphotographs in Figure 4a through 4e with Figure 4 shows that the viscosity of the cream gradually increased with cohesion and coalescence of the oil droplets (from point "a" to "b") and that the viscosity then sharply increased with the dispersion of water droplets into the oil phase which had expanded in the manner of an oil film (points "b" to "c"). The latter process constitutes a phase change from O/W type to W/O type. After the phase change, the viscosity decreased as the water droplets rapidly became extremely fine (points "d" to "e"). Finally, the cream was fully changed to a W/O emulsion (point "e"). At this point, no further change occurred in the viscosity.

These results show that the apparatus according to this invention is capable of positively determining emulsion phase change and of continuously measuring the dynamic characteristics of the phase change.

### Example 2

Chemically etched films were obtained by treating PTFE virgin films with a complex solution of naphthalene and metallic sodium in tetrahydrofuran and then rinsing them with water. While no substantial change in the surfaces of the films could be observed by a SEM, the color of the films changed from white to brown and the surface became hydrophilic. The films were attached to the opposed surfaces of the movable plate 1 and the pushing part 2 of the same apparatus as in Example 1.

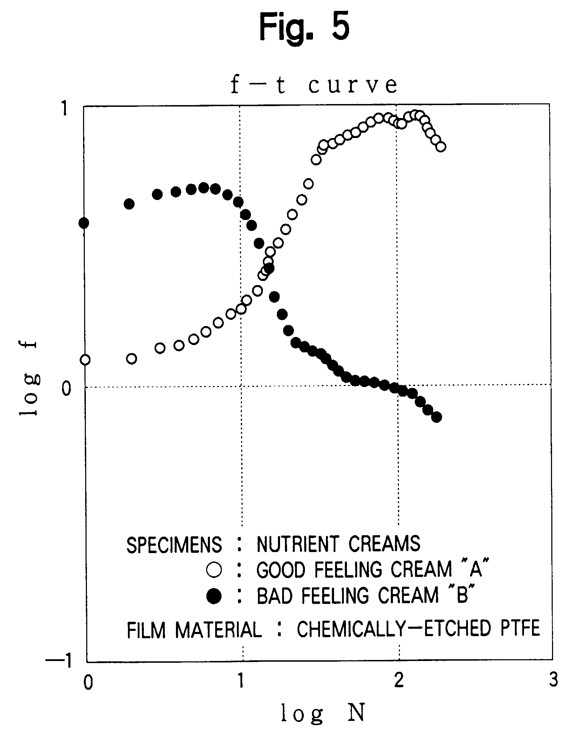

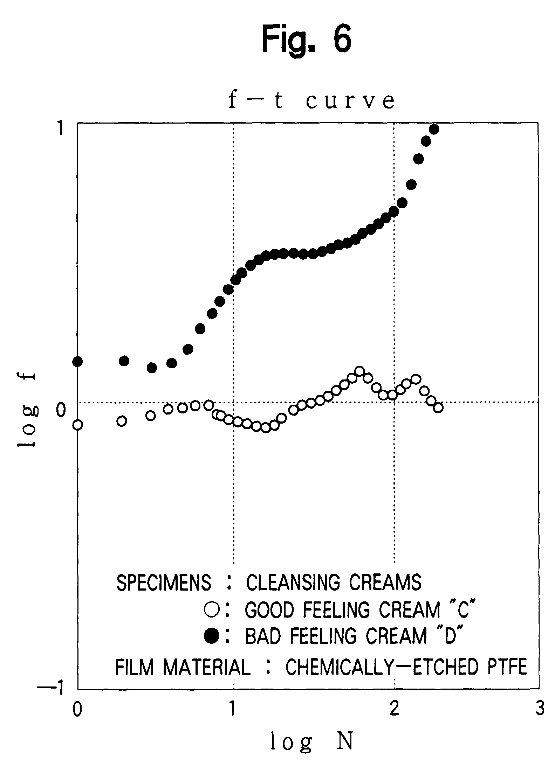

Two kinds of commercial nutrient creams "A" and "B" and two kinds of commercial cleansing creams "C" and "D" were tested under the same conditions as in Example 1. All of the creams were O/W type emulsions. The results obtained are shown in Figures 5 and 6, in which the two axes are graduated for the same parameters as in Figure 4.

On the other hand, tactile testing of the creams was conducted by five female panelers. The results obtained are shown in Table 1.

As is apparent from Figures 5 and 6 , there are significant differences in shear force between the creams "A"and "C" which the panelers rated us having a "good feeling" and the creams "B" and "D" which they rated as having a "bad feeling". It will be noted that the shear forces obtained by the tests are in close agreement with the panelers' feelings shown in Table 1. It can therefore be conducted that the results obtained by the apparatus agree well with the "feelings" rated by the panelers.

As shown above, the apparatus can accurately evaluate emulsion phase change and can therefore replace panelers in the evaluation of emulsions.

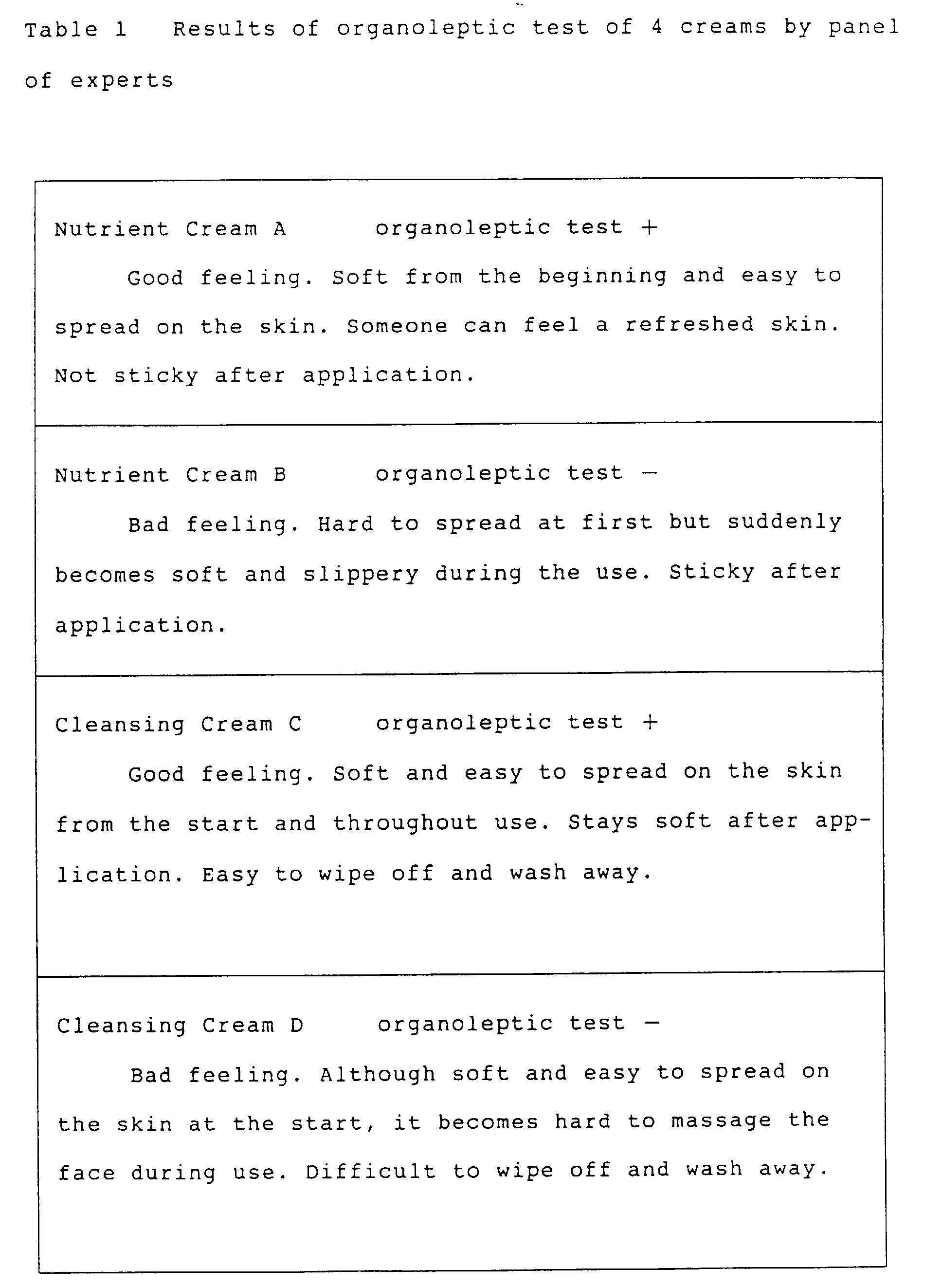

### Example 3

Chemically etched films were obtained by treating PTFE virgin films with a liquid ammonium solution of metallic sodium in tetrahydrofuran as in Example 2. In addition, porous films were obtained by heat stretching PTFE film. The chemically etched films, the porous films and the sputter-etched films obtained in Example 1 were attached to the opposed surfaces of the movable plate 1 and the pushing part 2 of the same apparatus as in Example 1. A commercial nutrient cream "A" was tested by the apparatus with air being blowing onto the cream. The surfaces of the pretreated PTFE films, the sputter-etched films and the porous films were fluorines, and that of the chemically etched film was carbons. The observation of the films showed that the surfaces of the pretreated PTFE films and the chemically etched films were generally smooth and that of the sputter-etched films had many fibres of 1.0 to 1.5 µ m length, and that of the porous films had many holes of about 2 µ m diameter.

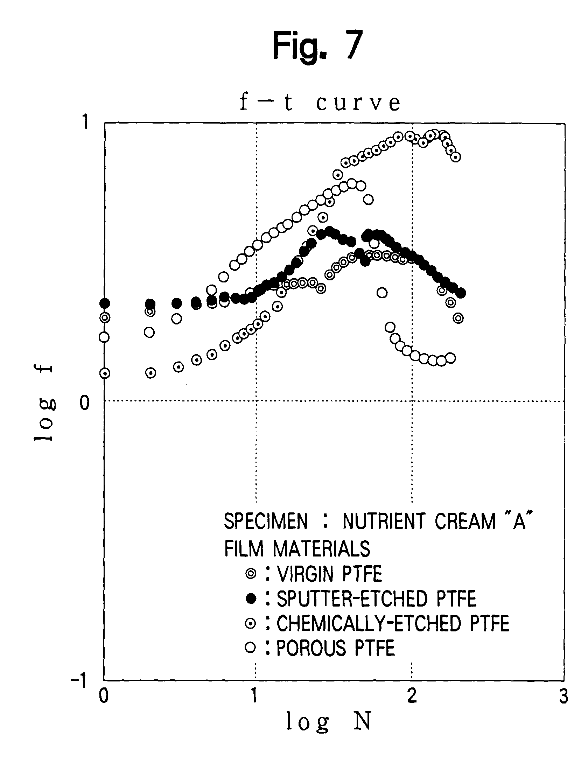

The test results obtained are shown in Figure 7. From the figure, it is found that the phase change behavior of the emulsion is influenced by the compositions and the structures of the surface materials. It also means that the phase change is influenced by the compositions and the cofigurations of the emulsion created at the initial stage of the phase change.

### Example 4

The sputter-etched films obtained in Example 1 were attached to the opposed surfaces of the disc 11 and the pushing part 2 of the apparatus shown in Figure 2 and a commercial nutrient cream "G" was tested by the apparatus at room temperature with air being blown onto the cream. The disc 11 was rotated at 9 revolutions per minute (rpm) and the number of rotations was 230.

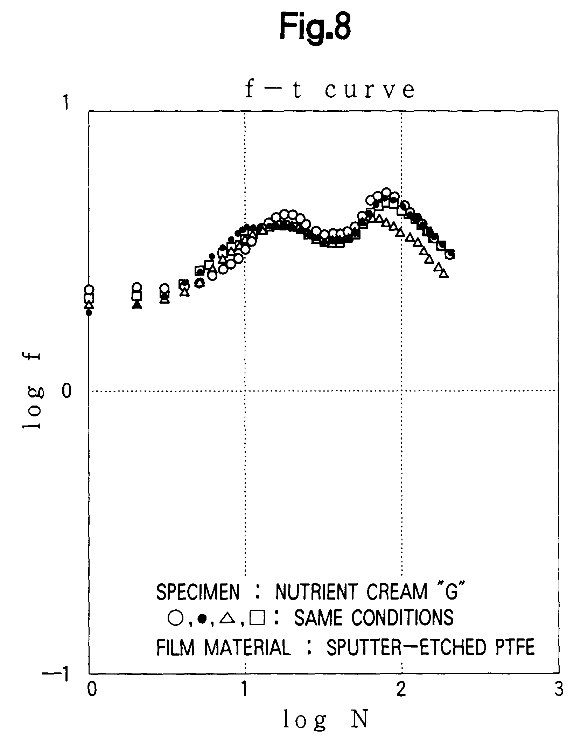

The results obtained are shown in Figure 8. In the figure, the horizontal axis is graduated for the logarithm (log N) of the number of rotations of the disc 11 and the vertical axis for the logarithm (log f) of the measured shearing force.

From Figure 8, it is apparent that the phase change behavior of an emulsion can also be determined by the apparatus of Figure 2.

### Example 5

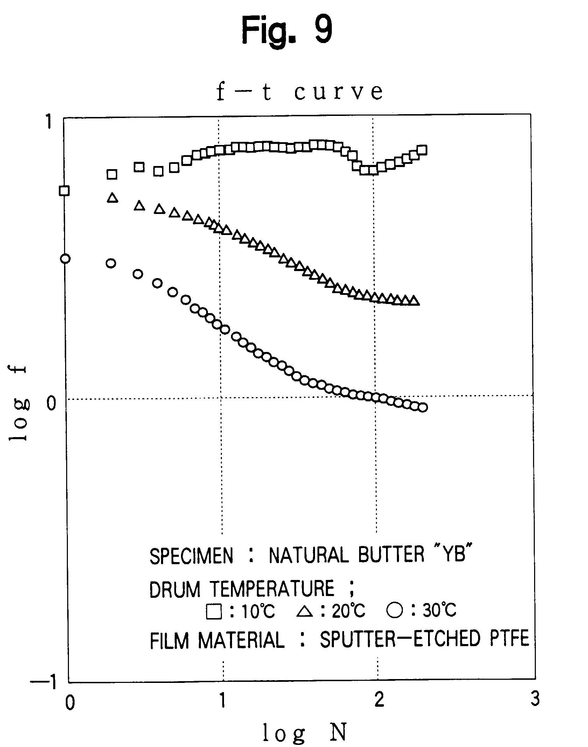

The sputter-etched films obtained in Example 1 were attached to the opposed surfaces of the drum 21 and the pushing part 2 of the apparatus shown in Figure 3 and a commercial natural butter "YB" was tested by the apparatus. The drum was rotated at 9 rpm and the number of rotations was 230. The test results are shown in Figure 9, in which the two axes represent the same parameters as in Figure 8.

The butter is a W/O type emulsion of water droplets dispersed in animal oil. The butter do not change phase in the temperature range of the test. Therefore the figure merely shows the changes in viscosity. As seen from Figure 9, there was no change in viscosity under 10°C while at over 20 °C the viscosity decreased with increasing number of rotations.

On the other hand, organoleptic test conducted by a panel on the butter under 20 °C and over 25°C showed it to be too soft to keep its shape over 25 °C . Comparing this result with the results in Figure 9 shows that the test results by the apparatus correspond to human feelings.

Although treated polymer films were attached to the opposed surfaces of the sliding part and the pushing part in the Example 1-5, the opposed surfaces contacting with the emulsion can be treated in response to their intended use and used for evaluating phase change of the emulsion.

The invention has thus been shown and described with reference to specific structures. However, it should be noted that the invention is in no way limited to the details of the illustrated structures but changes and modification may be made without departing from the scope of the appended claims.

## Claims

1. An apparatus for evaluating phase change of an emulsion comprising:
a sliding part (1, 11, 21) having a sliding surface to which the emulsion is applied,
a pushing part (2) which is movable toward the sliding surface to push against the emulsion on the sliding surface at a predetermined position, and
a sensor means (3) for continuously measuring a force acting on the pushing part (2) in the sliding direction during phase change of the emulsion and for evaluating the phase change of the emulsion from changes in the measured force
**characterized in that**
the top of the pushing part is attached to a vertically oriented coil spring (5).

2. An apparatus in accordance with claim 1, wherein films of a polymer substance are attached to at least one of said sliding surface (1,11,21) and said surface of the pushing part (2).

3. An apparatus in accordance with claim 2, wherein the films are chemically etched polymer films.

4. An apparatus in accordance with claim 2, wherein the films are sputter-etched polymer films.

5. An apparatus in accordance with claim 1, further comprising fan means (4) positioned laterally of the pushing part for blowing air onto the emulsion to evaporate its volatile components.

6. An apparatus in accordance with claim 1, further comprising temperature controlling means for controlling the temperature of the sliding part during the measurement between -20°C and 100°C.

7. An apparatus in accordance with claim 1, wherein the sliding part (1) is a reciprocating movable plate.

8. An apparatus in accordance with claim 1, wherein the sliding part (1) is a rotatable disc.

9. An apparatus in accordance with claim 1, wherein the sliding part (21) is a rotatable cylindrical drum.

10. A method for evaluating phase change of an emulsion comprising:
applying the emulsion to a sliding surface of a sliding part,
sliding the sliding part in parallel with the sliding surface, pushing a pushing part by elastically biasing using a vertically oriented coil spring the pushing part towards the sliding surface so that a surface of the pushing part opposed to the sliding surface is brought into contact with the emulsion on the sliding surface at a predetermined position during sliding movement of the sliding surface whereby the phase of the emulsion interposed between the sliding surface and the surface of the pushing part is changed to different phase thereof,
measuring a force which acts on the pushing part in the sliding direction, and
evaluating phase change of the emulsion from changes in the measured force.

## Patentansprüche

1. Vorrichtung zur Bestimmung der Phasenänderung einer Emulsion, umfassend:
ein gleitendes Teil (1, 11, 21) mit einer gleitenden Oberfläche, auf die eine Emulsion aufgetragen wird,
einem Drückerteil (2), das zu der gleitenden Oberfläche bewegbar ist, um gegen die Emulsion auf der gleitenden Oberfläche in einer vorgegebenen Position zu drücken, und
Sensormittel (3) für das kontinuierliche Messen einer auf das Drückerteil (2) in die Gleitrichtung wirkenden Kraft während der Phasenveränderung der Emulsion und für das Bestimmen der Phasenveränderung der Emulsion durch Änderungen in der gemessenen Kraft, dadurch gekennzeichnet, daß der Oberteil des Drückerteils an einer vertikal orientierten Schraubenfeder (5) angeordnet ist.

2. Vorrichtung nach Anspruch 1, dadurch gekennzeichnet, daß Filme aus einer polymeriden Substanz an mindestens einer der gleitenden Oberflächen (1, 11, 21) und der Oberfläche des Drückerteils (2) aufgebracht sind.

3. Vorrichtung nach Anspruch 2, dadurch gekennzeichnet, daß die Filme chemisch geätzte polymeride Filme sind.

4. Vorrichtung nach Anspruch 2, dadurch gekennzeichnet, daß die Filme sputtergeätzte polymeride Filme sind.

5. Vorrichtung nach Anspruch 1, dadurch gekennzeichnet, daß Ventilatormittel (4) vorgesehen sind, die seitlich des Drückerteils zum Blasen von Luft auf die Emulsion vorgesehen sind, um ihre flüchtigen Komponenten verdampfen zu lassen.

6. Vorrichtung nach Anspruch 1, dadurch gekennzeichnet, daß Temperatursteuermittel zum Steuern der Temperatur des gleitenden Teils während der Messungen zwischen -20°C und 100°C vorgesehen sind.

7. Vorrichtung nach Anspruch 1, dadurch gekennzeichnet, daß das gleitende Teil (1) eine hin- und herbewegbare Platte ist.

8. Vorrichtung nach Anspruch 1, dadurch gekennzeichnet, daß das gleitende Teil (1) eine drehbare Scheibe ist.

9. Vorrichtung nach Anspruch 1, dadurch gekennzeichnet, daß das gleitende Teil (21) eine drehbare zylindrische Trommel ist.

10. Verfahren zur Bestimmung der Phasenveränderung einer Emulsion, umfassend:
Aufbringen der Emulsion auf eine gleitende Oberfläche oder ein gleitendes Teil,
Verschieben des gleitenden Teils parallel zu der gleitenden Oberfläche, Schieben eines Drückerteils durch elastisches Spannen des Drückerteils unter Verwendung einer vertikal orientierten Schraubenfeder in Richtung der gleitenden Oberfläche, so daß eine Oberfläche des Drückerteils, die der gleitenden Oberfläche gegenüberliegt, in Kontakt mit der Emulsion auf der gleitenden Oberfläche an einer vorbestimmten Position während der gleitenden Bewegung der gleitenden Oberfläche gebracht wird, wobei die Phase der zwischen der gleitenden Oberfläche und der Oberfläche des Drückerteils angeordneten Emulsion zu einer unterschiedlichen Phase verändert wird, Messen einer Kraft, die auf das Drückerteil in die Gleitrichtung wirkt, und Bestimmen der Phasenänderung der Emulsion durch die Änderungen in der gemessenen Kraft.

## Revendications

1. Appareil d'évaluation d'un changement de phase d'une émulsion, comprenant :
une partie de glissement (1, 11, 21) ayant une surface de glissement à laquelle est appliquée l'émulsion,
une partie de poussée (2) qui est mobile vers la surface de glissement afin qu'elle pousse l'émulsion placée sur la surface de glissement à une position prédéterminée, et
un dispositif capteur (3) destiné à mesurer de façon continue une force agissant sur la partie de poussée (2) dans la direction de glissement au cours du changement de phase de l'émulsion et à évaluer constamment le changement de phase de l'émulsion d'après les changements de la force mesurée,
caractérisé en ce que :
la partie supérieure de la partie de poussée est fixée à un ressort hélicoïdal d'orientation verticale (5).

2. Appareil selon la revendication 1, dans lequel des films d'une substance polymère sont fixés à l'une au moins des surfaces de glissement (1, 11, 21) et de la partie de poussée (2).

3. Appareil selon la revendication 2, dans lequel les films sont des films polymères attaqués chimiquement.

4. Appareil selon la revendication 2, dans lequel les films sont des films polymères attaqués par pulvérisation.

5. Appareil selon la revendication 1, comprenant en outre un dispositif à ventilateur (4) placé latéralement par rapport à la partie de poussée et destiné à souffler de l'air sur l'émulsion pour provoquer l'évaporation de ses ingrédients volatils.

6. Appareil selon la revendication 1, comprenant en outre un dispositif de réglage de la température de la partie de glissement au cours de la mesure entre -20 °C et +100 °C.

7. Appareil selon la revendication 1, dans lequel la partie de glissement (1) est un plateau mobile alternatif.

8. Appareil selon la revendication 1, dans lequel la partie de glissement (1) est un disque rotatif.

9. Appareil selon la revendication 1, dans lequel la partie de glissement (21) est un tambour cylindrique rotatif.

10. Procédé d'évaluation d'un changement de phase d'une émulsion, comprenant :
l'application de l'émulsion à une surface de glissement d'une partie de glissement,
le glissement de la partie de glissement parallèlement à la surface de glissement,
la poussée d'une partie de poussée par rappel élastique, avec un ressort hélicoïdal d'orientation verticale, de la partie de poussée vers la surface de glissement afin qu'une surface de la partie de poussée opposée à la surface de glissement soit mise au contact de l'émulsion placée sur la surface de glissement à une position prédéterminée pendant le mouvement de glissement de la surface de glissement, si bien que la phase de l'émulsion placée entre la surface de glissement et la surface de la partie de poussée change et devient une phase différente,
la mesure d'une force qui agit sur la partie de poussée dans la direction de glissement, et
l'évaluation du changement de phase de l'émulsion d'après les changements de la force mesurée.
